# EUROPÄISCHE PATENTSCHRIFT

(11) **EP 1 091 935 B1**
(45) Veröffentlichungstag und Bekanntmachung des Hinweises auf die Patenterteilung: **21.04.2004**
(21) Anmeldenummer: 99929294.9
(22) Anmeldetag: 21.06.1999
(51) Int. Cl.: C07C 323/37, C07C 319/14

(54) **VERFAHREN ZUR HERSTELLUNG VON 2-NITRO-5-(PHENYLTHIO)-ANILINEN**
METHOD FOR PREPARING 2-NITRO-5-(PHENYLTHIO)-ANILINES
PROCEDE DE PREPARATION DE 2-NITRO-5-(PHENYLTHIO)-ANILINES

(30) Priorität: 01.07.1998 DE 19829357
(43) Veröffentlichungstag der Anmeldung: 18.04.2001
(73) Patentinhaber: Bayer Chemicals AG, 51368 Leverkusen (DE)
(72) Erfinder: GLOCK, Volker, D-47800 Krefeld (DE); STREICHER, Willi, D-47800 Krefeld (DE); ULLRICH, Friedrich-Wilhelm, D-51465 Bergisch Gladbach (DE)
(86) Internationale Anmeldenummer: PCT/EP1999/004296
(87) Internationale Veröffentlichungsnummer: WO 2000/001669

(56) Entgegenhaltungen:
- EP-A- 0 061 110
- CH-A- 619 458
- DE-A- 4 202 262
- US-A- 4 011 320
- CHEMICAL ABSTRACTS, Band 123, Nr. 7, 14. August 1995, Columbus, Ohio, US, Zusammen- fassungsnr. 83277c, HUANG, L. ET AL.: "Drugs against hydatidosis: syn- thesis of metabolites and analogs of albendazole", Seite 1036, linke Spalte, XP002900757 & Zhongguo Yiyao Gongye Zazhi 1995, 26(2), Seiten 55-59.
- CHEMICAL ABSTRACTS, Band 125, Nr. 5, 29. Juli 1996, Columubs, Ohio, US, Zusammenfassungs-Nr. 58055u, SKIBINSKI, A. ET AL.: "Pre- paration of dimethyl ester of N-(2-((methoxyacetyl)amino)- -4-(phenylthio)phenyl)carbon- -imidoyl-N,N'-bis-carbamic acid", Seite 1107, rechte Spalte, XP002900758 & Pol. J. Appl. Chem. 1995, 39(1), Seiten 91-94 (Eng).

## Beschreibung

2-Nitro-5-(phenylthio)-aniline sind wertvolle Zwischenprodukte zur Herstellung von Herbiziden (s. EP-A 61 110) zur Herstellung von Wirkstoffen zur Behandlung von Wurmkrankheiten (s. DE-A 2 332 398) und zur Herstellung von Wachstumförderungsmitteln für Haus- und Nutztiere (s. CH-A 619 458).

Zur Herstellung von 2-Nitro-5-(phenylthio)-anilinen kann man 5-Chlor-2-nitroaniline mit Thiophenolen z.B. in der Weise umsetzen, daß man zunächst mit einer Ölemulsion von Natriumhydrid in Dimethylformamid aus dem Thiophenpol das entsprechende Natriumthiophenolat bildet und dieses durch Zugabe eines 5-Chlor-2-nitroanilins zum Produkt umsetzt, das anschließend mit Wasser ausgefällt wird (s. DE-A 2 406 584).

Gemäß den DE-A 2 332 398 und DE-A 2 549 417 wurden in Dimethylformamid Thiophenol und 5-Chlor-2-nitroaniline in Gegenwart von Kaliumcarbonat ca. 7 Stunden unter Rückfluß gekocht. Nach wäßriger Aufarbeitung und Umkristallisieren wurde das Produkt in Ausbeuten von 77 bis 88 % erhalten. Bei nahezu gleicher Arbeitsweise, allerdings bei einer Reaktionszeit von nur 1 Stunde bei 100°C, wurde eine Rohausbeute von 91 % erreicht, das Material mußte aber noch umkristallisiert werden, um die nötige Qualität zu erreichen (s. J. Med. Chem. 18, 1164 (1975)).

Ebenfalls mit Kaliumcarbonat wird gemäß der EP-A 61 110 gearbeitet. Hier wurde in Dimethylsulfoxid bei 110°C 2-Methyl-6-nitro-3-phenylthio-anilin mit einer Ausbeute von 92 % hergestellt.

Nachteilig bei all diesen Verfahren ist, daß zur Produktisolierung die dipolare, aprotische Lösungsmittel enthaltenden Reaktionsgemische wäßrig aufgearbeitet werden müssen. Dies schafft ein stark mit organischen Lösungsmitteln belastetes Abwasser, das entsorgt werden muß. Darüber hinaus ist häufig die Ausbeute und/oder die Reinheit des erhaltenen Produkts unbefriedigend.

Zur Durchführung der Reaktion in Ethanol unter Rückflußbedingungen wurde Kaliumhydroxid als Base eingesetzt (S. CH-A 619 458 und Arch. Pharm. 316, 638 (1983)). Das Produkt wurde nach Abkühlen des Ansatzes abfiltriert und anschließend umkristallisiert, dabei wurden ebenfalls nicht befriedigende Ausbeuten von 80 bis 83 % erhalten.

Laut DE-A 4 202 262 wurde 5-Chtor-2-nitroanitin mit Thiophenol in einem Zweiphasensystem aus wäßriger Natronlauge und Toluol unter Mitwirkung von Tetrabutylammoniumbromid mit einer Ausbeute von 98 % hergestellt. Andere Phasentransferkatalysatoren lieferten schlechtere Ausbeuten. In diesem Verfahren werden Abwässer erzeugt, die mit den teilweise toxischen Phasentransferkatalysatoren belastet und wegen der Gegenwart der Phasentransferkatalysatoren nur sehr aufwendig zu reinigen sind.

Es besteht deshalb noch immer ein Bedürfnis nach einem Verfahren zur Herstellung von 2-Nitro-5-phenyl-thioanilinen, das einerseits mit guten Ausbeuten durchführbar ist, aber andererseits keine besonderen ökologischen Probleme verursacht.

Es wurde nun ein Verfahren zur Herstellung von 2-Nitro-5-(phenylthio)-anilinen der Formel (I) 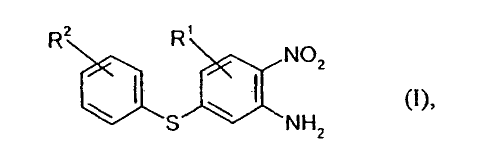 in der
- R¹: für Wasserstoff, C₁-C₈-Alkyl, C₃-C₈-Cycloalk-yl, C₁-C₈-Alkoxy oder Halogen und
- R²: für Wasserstoff, C₁-C₈-Alkyl, C₃-C₈-Cycloalkyl, C₁-C₈-Alkoxy, Halogen oder gegebenenfalls mit C₁-C₈-Alkyl, C₁-C₈-Alkoxy oder Halogen substituiertes C₆-C₁₀-Aryl stehen,
durch Umsetzung von 5-Chlor-2-nitroanilinen der Formel (II) 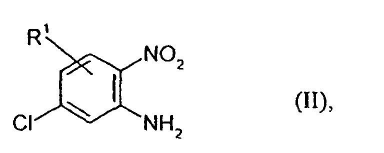 in der
- R¹: die bei Formel (I) angegebene Bedeutung hat,
mit Thiophenolen der Formel (III) 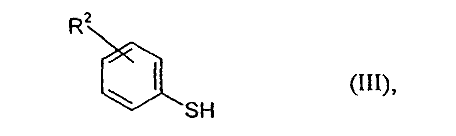 in der
- R²: die bei Formel (I) angegebene Bedeutung hat,
das dadurch gekennzeichnet ist, daß man ein 5-Chlor-2-nitroanilin der Formel (II) in einem Lösungsmittel mit einem Thiophenol der Formel (III) und Ammoniak umsetzt.

In den Formeln (I) und (II) steht R¹ vorzugsweise für Wasserstoff, C₁-C₄-Alkyl oder Chlor, besonders bevorzugt für Wasserstoff, Methyl oder Chlor.

In das erfindungsgemäße Verfahren einsetzbare 5-Chlor-2-nitroaniline der Formel (II) und Thiophenole der Formel (III) können, soweit sie nicht im Handel erhältlich sind, auf bekannte Weise oder analog dazu erhalten werden.

Bei einer bevorzugten Ausführungsform des erfindungsgemäßen Verfahrens stellt man das jeweilige 5-Chlor-2-nitroanilin durch Chlor-Amin-Austausch aus dem entsprechenden 2,4-Dichlomitrobenzol mit Ammoniak her (s. z.B. J. Med. Chem. 35, 4455 (1992) und DE-A 3 431 827)) und führt dann das erfindungsgemäße Verfahren ohne Zwischenisolierung des gebildeten 5-Chlor-2-nitroanilins durch Zugabe des Thiophenols und gegebenenfalls Nachdosierung von Ammoniak im gleichen Reaktionsgefäß durch. Im Prinzip ist beim erfindungsgemäßen Verfahren die Reihenfolge des Zusammenfügens der Reaktanden unkritisch. Bevorzugt versetzt man das 5-Chlor-2-nitroanilin der Formel (II) gelöst in einem Lösungsmittel mit Ammoniak und fügt dann ein Thiophenol der Formel (III) zu. Man kann die Reaktanden auch in beliebiger anderer Reihenfolge zusammengeben. Man kann sie auch dem Reaktionsgemisch simultan zudosieren.

In den Formeln (I) und (III) steht R² vorzugsweise für Wasserstoff oder C₁-C₄-Alkyl, besonders bevorzugt für Wasserstoff.

Als Lösungsmittel für das erfindungsgemäße Verfahren kommen praktisch alle üblichen Lösungsmittel infrage, z.B. anorganische wie Wasser oder wasserfreier Ammoniak und organische wie Alkohole, Ether, Kohlenwasserstoffe, Aromaten, Chloraromaten und dipolar-aprotische Lösungsmittel. Als Einzelbeispiele für organische Lösungsmittel seien genannt: Methanol, Ethanol, n-Propanol, i-Propanol, n-Butanol, i-Butanol, sec.-Butanol, tert.-Butanol, Glykol, Diethylether, Methyl-tert.-butylether, Tetrahydrofuran, Dimethylformamid, N-Methylpyrrolidon, Toluol und Chlorbenzol. Bevorzugt sind unpolare und wenig polare Lösungsmittel wie Alkohole, Ether, Kohlenwasserstoffe, Aromaten und Chloraromaten, insbesondere Methanol, Isopropanol, Isobutanol, Toluol und Chlorbenzol.

Der Einsatz von Phasentransferkatalysatoren ist nicht nötig.

Der Ammoniak kann in technischen Qualitäten eingesetzt werden.

Bezogen auf 1 Mol des eingesetzten 5-Chlor-2-nitroanilins kann man beispielsweise 0,5 bis 2 Mol, insbesondere 1 bis 1,2 Mol eines Thiophenols und 1 bis 30 Mol, insbesondere 2 bis 15 Mol Ammoniak einsetzen.

Das erfindungsgemäße Verfahren kann z.B. bei Temperaturen im Bereich von 20 bis 140°C durchgeführt werden. Bevorzugt arbeitet man bei 40 bis 100°C. Während der Durchführung des erfindungsgemäßen Verfahrens können z.B. Drucke im Bereich von 1 bis 20 bar vorliegen. Vorzugsweise arbeitet man bei 3 bis 12 bar, insbesondere unter dem sich bei den angewendeten Reaktionsbedingungen in einem geschlossenem Reaktionsgefäß von selbst einstellenden Druck. Es ist vorteilhaft, insbesondere gegen Ende der Reaktion, die Temperatur unter 80°C und den Druck unter 10 bar zu halten.

Man kann das erfindungsgemäße Verfahren z.B. in der Weise durchführen, daß man zunächst das 5-Chlor-2-nitroanilin der Formel (II) zusammen mit dem Lösungsmittel in einem Autoklaven vorlegt, dann auf die gewünschte Reaktionstemperatur erhitzt, dann Ammoniak zufügt und anschließend das Thiophenol der Formel (III) zudosiert, z.B. im Verlaufe von 0,5 bis 5 Stunden. Es ist vorteilhaft, während der Zudosierung des Thiophenols Ammoniak nachzudosieren, z.B. so, daß der Druck, der vor Beginn der Zudosierung des Thiophenols geherrscht hat, innerhalb einer Abweichungsbreite von ± 20 % erhalten bleibt. Nach Beendigung der Thiophenolzudosierung kann man noch einige Zeit, z.B. 1 bis 20 Stunden unter den o.a. Druck- und Temperaturbedingungen, insbesondere bei Temperaturen unter 80°C nachrühren.

Die Aufarbeitung des dann vorliegenden Reaktionsgemisches ist einfach. Häufig, insbesondere beim Einsatz von unpolaren oder wenig polaren Lösungsmitteln, liegt nach Abkühlung des Autoklaven eine Suspension vor, die das hergestellte 2-Nitro-5-(phenylthio)-anilin der Formel (I) in fester Form enthält. Dann ist es i.a. ausreichend, das Reaktionsgemisch abzufiltrieren und den festen Rückstand beispielsweise mit Wasser zu waschen. Gewünschtenfalls kann man aus dem gebildeten Ammoniumchlorid durch Zusatz einer starken Base, z.B. wäßriger Natronlauge, Ammoniak zurückgewinnen. Beim Einsatz dipolarer, aprotischer Lösungsmittel ist es vorteilhaft, dem Reaktionsgemisch vor der Abtrennung des hergestellten Produkts Wasser hinzuzufügen.

Mit dem erfindungsgemäßen Verfahren erhält man 2-Nitro-5-(phenylthio)-aniline der Formel (I) i.a. in Ausbeuten von über 88 % d.Th. und Reinheiten von über 90 Gew.-%. Häufig liegen die Ausbeuten bei über 92 % d.Th., z.T. über 95 % d.Th. und die Reinheiten bei über 94 Gew.-%.

Es ist ausgesprochen überraschend, daß mit dem erfindungsgemäßen Verfahren so gute Ergebnisse auf einfache Weise erzielbar sind, ohne daß zwangsweise besondere ökologische Probleme auftreten, da Ammoniak selbst ein gutes Nucleophil ist und an Aromaten gebundenes Chlor gegen die Amino-Gruppc austauschen kann. Überraschenderweise tritt eine derartige Reaktion mit den eingesetzten Verbindungen der Formel (II) jedoch praktisch nicht auf. Beim erfindungsgemäßen Verfahren wird dagegen in unerwarteter Weise dagegen trotz der Gegenwart von Ammoniak das in den Verbindungen der Formel (II) vorhandene Chlor selektiv durch den Thiophenolrest ersetzt.

Die Ökologie ist besonders günstig, wenn man unpolare oder wenig polare Lösungsmittel einsetzt, da diese nur in geringen Mengen ins Abwasser gelangen. Bisher hat man dagegen den Einsatz dipolarer, aprotischer Lösungsmittel oder den Einsatz von Phasentransfer-Katalysatoren (in Kombination mit wenig polaren Lösungsmitteln) für unabdingbar gehalten. Dipolare, aprotische Lösungsmittel und wenig polare Lösungsmittel in Gegenwart von Phasentransferkatalysatoren gelangen jedoch in großen Mengen ins Abwasser.

### Beispiele

### Beispiel 1

In einem 1 l Stahlautoklaven wurden 200 g 5-Chlor-2-nitroanilin (87,3 gew.-%ig) in 200 ml Chlorbenzol suspendiert. Dann wurde der Ansatz auf 60°C erwärmt und 45,9 g Ammoniak in den Autoklaven eingepumpt, wobei sich ein Druck von 4 bar einstellte. Anschließend wurden bei dieser Temperatur 123 g Thiophenol (98 gew.-%ig) innerhalb von 2 Stunden in den Autoklaven gepumpt. Gleichzeitig wurde der Druck durch Nachdosieren von Ammoniak konstant auf 4 bar gehalten. Nach 6 Stunden wurde der Autoklav abgekühlt und entspannt. Der Ansatz wurde mit 195 g Natronlauge (23 gew.-%ig) versetzt und auf 90°C erwärmt. Bei 90°C wurden dann die Phasen getrennt. Die organische Phase wurde auf Raumtemperatur abgekühlt, sie enthielt 234 g 2-Nitro-5-(phenylthio)-anilin, was einer Ausbeute von 92,2 % d.Th. entspricht. Eine Umkristallisation war nicht erforderlich.

### Beispiel 2

In einem Autoklaven wurden 255 g 5-Chlor-2-nitroanilin (78,3 gew.-%ig) in 250 ml Isopropanol suspendiert. Man erwärmte den Ansatz auf 60°C und pumpte 95,7 g Ammoniak in den Autoklaven, wobei sich ein Druck von 9 bar einstellte. Anschließend wurden bei dieser Temperatur 161 g Thiophenol (98 gew.-%ig) im Verlaufe von 1,5 Stunden in den Autoklaven gepumpt. Gleichzeitig hielt man den Druck durch Nachdosieren von Ammoniak auf 9 bar. Nach 6 Stunden wurde der Autoklav auf Raumtemperatur abgekühlt und entspannt. Der Inhalt des Autoklaven wurde abfiltriert, der Autoklav mit Isopropanol nachgespült und die Spülflüssigkeit ebenfalls abfiltriert. Die gesammelten Rückstände wurden mit Wasser gewaschen und getrocknet. Man erhielt 298,6 g 2-Nitro-5-(phenylthio)-anilin in Form eines gelben Pulvers mit einem Gehalt von 91,2 Gew.-%. Das entspricht einer Ausbeute von 96,4 % d.Th.

### Beispiel 3

In einem Autoklaven wurden 200 g 5-Chlor-2-nitroanilin (79,2 gew.-%ig) in 200 ml Isobutanol suspendiert. Man erwärmte den Ansatz auf 60°C und pumpte 26 g Ammoniak dazu, wobei sich ein Druck von 4 bar einstellte. Anschließend wurden bei dieser Temperatur 126 g Thiophenol (98 gew.-%ig) im Verlaufe von 1,5 Stunden in den Autoklaven gepumpt. Gleichzeitig wurde der Druck durch Nachdosieren von Ammoniak bei 4 bar gehalten. Nach 6 Stunden wurde der Autoklav auf Raumtemperatur abgekühlt und entspannt. Der Inhalt des Autoklaven wurde abfiltriert, der Autoklav mit Isobutanol nachgespült und die Spülflüssigkeit ebenfalls abfiltriert. Die vereinigten Rückstände wurden mit Wasser gewaschen und getrocknet. Man erhielt 240,6 g 2-Nitro-5-(phenylthio)-anilin in Form eines gelben Pulvers mit einem Gehalt von 90,0 Gew.-%. Das entspricht einer Ausbeute von 95,8 % d.Th.

### Beispiel 4

In einem Autoklaven wurden 358,8 g 5-Chlor-2-nitroanilin (79,2 gew.-%ig) in 410 ml Toluol suspendiert. Man erwärmte den Ansatz auf 60°C und pumpte 98 g Ammoniak in den Autoklaven, wobei sich ein Druck von 9 bar einstellte. Anschließend wurden bei dieser Temperatur 253,5 g Thiophenol (98 gew.-%ig) im Verlaufe von 2 Stunden in den Autoklaven gepumpt. Gleichzeitig wurde der Druck durch Nachdosieren von Ammoniak konstant auf 9 bar gehalten. Nach 6 Stunden wurde der Autoklav auf Raumtemperatur abgekühlt und entspannt. Das Reaktionsgemisch enthielt 384,5 g 2-Nitro-5-(phenylthio)-anilin, was einer Ausbeute von 94,8 % d.Th. entspricht.

### Beispiel 5

Es wurde verfahren wie in Beispiel 4, jedoch wurde als Lösungsmittel eine entsprechende Menge Methanol eingesetzt und das Produkt in einer Ausbeute von 97,7 % d.Th. erhalten.

### Beispiel 6

Es wurde verfahren wie in Beispiel 4, jedoch wurde als Lösungsmittel eine entsprechende Menge Dimethylformamid eingesetzt und das Produkt nach Zusatz von Wasser in nahezu quantitative Reaktionsausbeute erhalten.

### Beispiel 7

Es wurde verfahren wie in Beispiel 4, jedoch wurde als Lösungsmittel eine entsprcchende Menge Wasser eingesetzt und das Produkt in einer Reaktionsausbeute von 98 % d.Th. erhalten.

### Beispiel 8

In einem Autoklaven wurden 200 ml Isopropanol vorgelegt und 196 g 2,4-Dichlornitrobenzol (98 gew.-%ig) zugegeben. Der Autoklav wurde verschlossen und auf 120°C geheizt. Bei dieser Temperatur wurde Ammoniak eingepumpt, bis ein Druck von 30 bar erreicht war. Durch Nachdosieren von Ammoniak während der Reaktion wurde der Druck in den folgenden 12 Stunden auf 30 bar gehalten. Anschließend kühlte man den Ansatz auf 60°C ab und dosierte im Verlaufe von 2 h 118 g Thiophenol (98 gew.-%ig) dazu. Anschließend wurde 6 Stunden nachgerührt. Der Ansatz wurde auf Raumtemperatur abgekühlt und entspannt. Die Produktsuspension wurde abgesaugt, der Feststoff zunächst mit Isopropanol, dann mit Wasser gewaschen und getrocknet. Man erhielt 232,2 g 2-Nitro-5-(phenylthio)-anilin mit einem Gehalt von 94,9 Gew.-%. Das entsprach einer Ausbeute von 89,5 % d.Th.

## Patentansprüche

1. Verfahren zur Herstellung von 2-Nitro-5-(phenylthio)-anilinen der Formel (I) 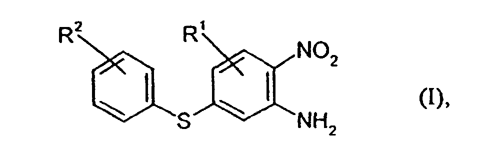 in der
R¹ für Wasserstoff, C₁-C₈-Alkyl, C₃-C₈-Cycloalkyl, C₁-C₈-Alkoxy oder Halogen und
R² für Wasserstoff, C₁-C₈-Alkyl, C₃-C₈-Cycloalkyl, C₁-C₈-Alkoxy, Halogen oder gegebenenfalls mit C₁-C₈-Alkyl, C₁-C₈-Alkoxy oder Halogen substituiertes C₆-C₁₀-Aryl stehen,
durch Umsetzung von 5-Chlor-2-nitroanilinen der Forrriel (II) 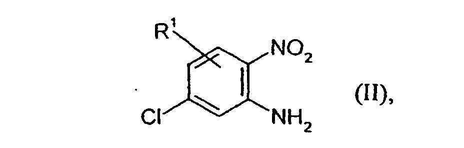 in der
R¹ die bei Formel (I) angegebene Bedeutung hat,
mit Thiophenolen der Formel (III) 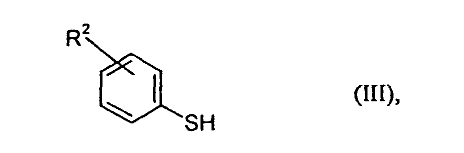 in der
R² die bei Formel (I) angegebene Bedeutung hat,
**dadurch gekennzeichnet, daß** man ein 5-Chlor-2-nitroanilin der Formel (II) in einem Lösungsmittel mit einem Thiophenol der Formel (III) und Ammoniak umsetzt.

2. Verfahren nach Anspruch 1, **dadurch gekennzeichnet**, das in den Formeln R¹ für Wasserstoff, C₁-C₄-Alkyl oder Chlor und R² für Wasserstoff oder C₁-C₄-Alkyl stehen.

3. Verfahren nach Ansprüchen 1 und 2, **dadurch gekennzeichnet, daß** man das 5-Chlor-2-nitroanilin der Formel (II) durch Chlor-Amin-Austausch aus dem entsprechenden 2,4-Dichlornitrobenzol mit Ammoniak hergestellt und dann ohne Zwischenisolierung des gebildeten 5-Chlor-2-nitroanilins durch Zugabe eines Thiophenols der Formel (III) im gleichen Reaktionsgefäß ein 2-Nitro-5-(phenylthio)-anilin der Formel (I) herstellt.

4. Verfahren nach Ansprüchen 1 bis 3, **dadurch gekennzeichnet, daß** man als Lösungsmittel Wasser, wasserfreien Ammoniak, Alkohole, Ether, Kohlenwasserstoffe, Aromaten, Chloraromaten oder dipolar-aprotische Lösungsmittel einsetzt.

5. Verfahren nach Ansprüchen 1 bis 4, **dadurch gekennzeichnet, daß** man bezogen auf 1 Mol des eingesetzten 5-Chlor-2-nitroanilins 0,5 bis 2 Mole eines Thiophcnols und 2 bis 15 Mole Ammoniak einsetzt.

6. Verfahren nach Ansprüchen 1 bis 5, **dadurch gekennzeichnet, daß** man es bei 20 bis 140°C durchführt.

7. Verfahren nach Ansprüchen 1 bis 6, **dadurch gekennzeichnet, daß** der Druck im Bereich 1 bis 20 bar liegt.

8. Verfahren nach Ansprüchen 1 bis 7, **dadurch gekennzeichnet, daß** man unter dem sich bei den angewendeten Reaktionsbedingungen in einem geschlossenen Reaktionsgefäß von selbst einstellenden Druck arbeitet.

9. Verfahren nach Ansprüchen 1 bis 8, **dadurch gekennzeichnet, daß** man zunächst das 5-Chlor-2-nitroanilin der Formel (II) zusammen mit dem Lösungsmittel in einem Autoklaven vorlegt, dann auf die gewünschte Reaktionstemperatur erhitzt, dann Ammoniak zufügt und anschließend das Thiophenol der Formel (III) zudosiert.

10. Verfahren nach Ansprüchen 1 bis 9, **dadurch gekennzeichnet, daß** man das nach Beendigung der Reaktion vorliegende Gemisch aufarbeitet, indem man es abkühlt, gegebenenfalls mit Wasser versetzt, abfiltriert und den Rückstand wäscht.

## Claims

1. Process for preparing 2-nitro-5-(phenylthio)-anilines of the formula (I) 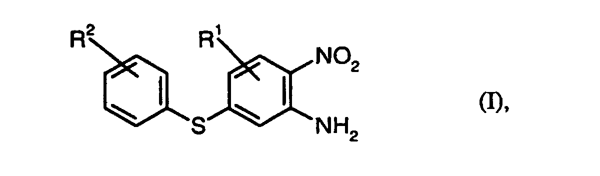 in which
R¹ represents hydrogen, C₁-C₈-alkyl, C₃-C₈-cycloalkyl, C₁-C₈-alkoxy or halogen and
R² represents hydrogen, C₁-C₈-alkyl, C₃-C₈-cycloalkyl, C₁-C₈-alkoxy, halogen or optionally C₁-C₈-alkyl-, C₁-C₈-alkoxy- or halogen-substituted C₆-C₁₀-aryl,
by reacting 5-chloro-2-nitroanilines of the formula (II) 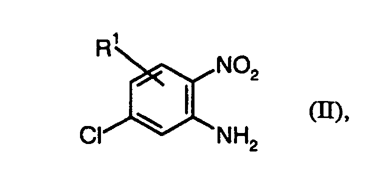 in which
R¹ is as defined under formula (I)
with thiophenols of the formula (III) 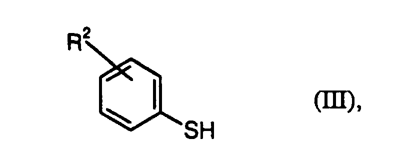 in which
R² is as defined under formula (I),
**characterized in that** a 5-chloro-2-nitroaniline of the formula (II) is reacted in a solvent with a thiophenol of the formula (III) and ammonia.

2. Process according to Claim 1, **characterized in that** in the formulae R¹ represents hydrogen, C₁-C₄-alkyl or chorine and R² represents hydrogen or C₁-C₄-alkyl.

3. Process according to Claims 1 and 2, **characterized in that** the 5-chloro-2-nitroaniline of the formula (II) is prepared by chlorine-amine exchange from the corresponding 2,4-dichloronitrobenzene using ammonia, and a 2-nitro-5-(phenylthio)-aniline of the formula (I) is then prepared without intermediate isolation of the 5-chloro-2-nitrolaniline formed by adding a thiophenol of the formula (III), in the same reaction vessel.

4. Process according to Claims 1 to 3, **characterized in that** the solvents used are water, anhydrous ammonia, alcohols, ethers, hydrocarbons, aromatics, chloroaromatics or dipolar aprotic solvents.

5. Process according to Claims 1 to 4, **characterized in that**, based on 1 mole of the 5-chloro-2-nitroaniline used, from 0.5 to 2 mols of a thiophenol and from 2 to 15 mols of ammonia are employed.

6. Process according to Claims 1 to 5, **characterized in that** the process is carried out at from 20 to 140°C.

7. Process according to Claims 1 to 6, **characterized in that** the pressure is in the range from 1 to 20 bar.

8. Process according to Claims 1 to 7, **characterized in that** the reaction is carried out under the autogenous pressure that becomes established in a sealed reaction vessel under the reaction conditions used.

9. Process according to Claims 1 to 8, **characterized in that** the 5-chloro-2-nitroaniline of the formula (II) is initially charged with the solvent in an autoclave, the mixture is then heated to the desired reaction temperature, ammonia is then added and the thiophenol of the formula (III) is then metered in.

10. Process according to Claims 1 to 9, **characterized in that** the mixture that is present after the reaction has ended is worked up **in that** it is cooled, and if appropriate mixed with water and filtered off, and the residue is washed.

## Revendications

1. Procédé pour la préparation des 2-nitro-5-(phénylthio)-anilines de formule (I) 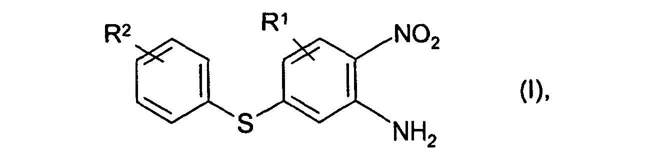 dans laquelle
R¹ représente l'hydrogène, un groupe alkyle en C₁-C₈, cycloalkyle en C₃-C₈, alcoxy en C₁-C₈ ou un halogène et
R² représente l'hydrogène, un groupe alkyle en C₁-C₈, cycloalkyle en C₃-C₈, alcoxy en C₁-C₈, un halogène ou un groupe aryle en C₆-C₁₀ portant éventuellement des substituants alkyle en C₁-C₈, alcoxy en C₁-C₈ ou halogéno,
par réaction de 5-chloro-2-nitranilines de formule (II) 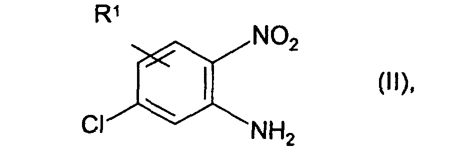 dans laquelle
R¹ a les significations indiquées en référence à la formule (I),
avec des thiophénols de formule (III) 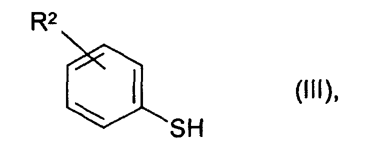 dans laquelle
R² a les significations indiquées en référence à la formule (I),
ce procédé **se caractérisant en ce que** l'on fait réagir une 5-chloro-2-nitraniline de formule (II), dans un solvant avec un thiophénol de formule (III) et de l'ammoniac.

2. Procédé selon la revendication 1, **caractérisé en ce que**, dans les formules, R¹ représente l'hydrogène, un groupe alkyle en C₁-C₄ ou le chlore et R² l'hydrogène ou un groupe alkyle en C₁-C₄.

3. Procédé selon les revendications 1 et 2, **caractérisé en ce que** l'on prépare la 5-chloro-2-nitraniline de formule (II) à partir du 2,4-dichloronitrobenzène correspondant par échange chlore-amine à l'aide de l'ammoniac puis, sans isoler la 5-chloro-2-nitraniline formée, on ajoute un thiophénol de formule (III) ce qui donne, dans le même récipient de réaction, une 2-nitro-5-(phénylthio)aniline de formule (I).

4. Procédé selon les revendications 1 à 3, **caractérisé en ce que** le solvant utilisé est l'eau, l'ammoniac anhydre, un alcool, un éther, un hydrocarbure, un dérivé aromatique, un dérivé aromatique chloré ou un solvant aprotonique dipolaire.

5. Procédé selon les revendications 1 à 4, **caractérisé en ce que**, pour une mole de la 5-chloro-2-nitraniline mise en oeuvre, on utilise 0,5 à 2 mol d'un thiophénol et 2 à 15 mol d'ammoniac.

6. Procédé selon les revendications 1 à 5, **caractérisé en ce que** l'on opère à des températures de 20 à 140°C.

7. Procédé selon les revendications 1 à 6, **caractérisé en ce que** l'on opère à des pressions dans l'intervalle de 1 à 20 bar.

8. Procédé selon les revendications 1 à 7, **caractérisé en ce que** l'on opère à la pression qui s'établit d'elle-même dans un récipient de réaction fermé dans les conditions de réaction observées.

9. Procédé selon les revendications 1 à 8, **caractérisé en ce que** l'on introduit d'abord dans un autoclave la 5-chloro-2-nitraniline de formule (II) avec le solvant, on chauffe à la température de réaction voulue puis on ajoute l'ammoniac et finalement le thiophénol de formule (III).

10. Procédé selon les revendications 1 à 9, **caractérisé en ce que** le mélange obtenu après la réaction est refroidi, le cas échéant additionné d'eau, filtré, et le résidu lavé.
